# EUROPEAN PATENT APPLICATION

(11) **EP 4 635 979 A1**
(43) Date of publication of application: **22.10.2025**
(21) Application number: 23927256.0
(22) Date of filing: 28.12.2023
(51) Int. Cl.: C07K 14/78, C12N 15/12, C12N 15/70, C12N 1/21, C07K 1/14, C07K 1/22, A61K 38/39, A61K 8/65, A61Q 19/00, A61P 29/00, A23L 33/18, A23L 29/00, C12R 1/19

(54) **LOW-MOLECULAR-WEIGHT COLLAGEN HAVING 164.88° TRIPLE-HELIX STRUCTURE**

(30) Priority: 16.03.2023 CN 202310268735; 16.05.2023 CN 202310553965; 01.08.2023 WO PCT/CN2023/110578
(71) Applicant: Shanxi Jinbo Bio-Pharmaceutical Co., Ltd., Taiyuan, Shanxi 030032 (CN)
(72) Inventor: WANG, Lingling, Taiyuan, Shanxi 030032 (CN); ZHANG, Yongjian, Taiyuan, Shanxi 030032 (CN); DENG, Pengjun, Taiyuan, Shanxi 030032 (CN); HE, Zhenrui, Taiyuan, Shanxi 030032 (CN); CHEN, Chuanxiu, Taiyuan, Shanxi 030032 (CN)
(74) Representative: Cabinet Becker et Associés
(86) International application number: PCT/CN2023/142879
(87) International publication number: WO 2024/187916

(57) **Abstract**

Provided is a low-molecular-weight collagen having a 164.88° triple-helix structure. The present invention relates to small-molecule collagen, a preparation method therefor, and a use thereof. The collagen has an amino acid sequence represented by SEQ ID NO: 1 or a variant amino acid sequence after mutation of the amino acid sequence. The variant amino acid sequence retains the function of the amino acid sequence represented by SEQ ID NO: 1. The collagen has a short amino acid sequence and good transdermal absorption performance.

## Description

The present application claims the priority benefits of the Chinese patent application No. 202310268735.1 titled "Low-Molecular-Weight Collagen Having 164.88° Triple-Helix Structure" filed on March 16, 2023; the Chinese patent application No. 202310553965.2 titled "Low-Molecular-Weight Collagen Having 164.88° Triple-Helix Structure" filed on May 16, 2023; and the PCT patent application No: PCT/CN2023/110578, titled "Low-Molecular-Weight Collagen Having 164.88° Triple-Helix Structure" filed on August 1, 2023. The aforementioned applications are incorporated herein by reference.

### Technical field

The present application relates to the field of collagen, specifically to small-molecule collagen, a preparation method thereof and a use thereof.

### Background of the Invention

Collagen is a class of proteins widely distributed in human connective tissues and is the most abundant protein in the human body, accounting for 25%-35% of the total protein content. Its main functions are reflected in aspects such as maintaining the extracellular environment, supporting the normal physiological functions of tissues and organs, and repairing body damage. Collagen is a natural biological resource with unparalleled biotissue compatibility, cell-supporting elasticity, and degradability compared to other high-molecular materials, making it widely applicable in industries such as pharmaceutics and cosmetics.

Natural collagen molecules can form a unique superhelical structure, which is a left-handed helix with three amino acid residues as the basic repeating unit, typically Gly-X-Pro. Glycine (Gly) is essential for hydrogen bond formation in collagen, and its lack of side chains allows for tight packing of collagen, which can maintain skin tension and elasticity. As people age, collagen in the dermis degrades and ages, leading to dry skin and wrinkles. To prevent skin aging, researchers have studied supplementing collagen to delay the skin aging process.

Currently, collagen is known to be a high-molecular-weight protein. In terms of molecular weight, it is difficult to be absorbed through the skin; it can only enter the dermis through micro-wounds in damaged skin, hair follicles, and sweat glands to supplement collagen. Therefore, how to promote the transdermal absorption of collagen has become a research focus in prior studies.

In recent years, with the widespread application of genetic engineering technologies, researchers have developed various types of recombinant collagens. For example, short amino acid sequences derived from natural human collagens can be selected to construct recombinant collagens. These recombinant collagens exhibit advantages such as low immunogenicity, high biological activity, and good stability. Theoretically, the shorter the amino acid sequence of such recombinant collagen, the better its transdermal absorption performance; however, shorter sequences are not necessarily optimal. In the prior art, there is still a lack of theoretical guidance on how to design short amino acid sequences to achieve superior transdermal absorption performance in constructed recombinant collagens.

There remains a need in the art for new small-molecule collagens.

### Summary of the invention

In order to meet the needs in the prior art, the inventors have discovered a new collagen with a molecular weight of approximately 2814.07 Da, which can penetrate the dermis of skin to reach subcutaneous tissues. Upon research, data show that the collagen of the present application has a skin penetration rate of more than 85% after being applying to the skin for 12h, showing excellent collagen transdermal absorption effect. This effect is significantly superior to that of high-molecular-weight collagens currently available on the market.

In one aspect, the present application provides a collagen comprising the following amino acid sequence:
(1) an amino acid sequence shown in SEQ ID NO: 1;
(2) an amino acid sequence having 50%, 60%, 70%, 75%, 80%, 85%, 90%, 95%, 96%, 97%, 98%, or 99% sequence identity to the amino acid sequence shown in SEQ ID NO: 1; or
(3) an amino acid sequence in which a mutation of one or more amino acid residues is made in the amino acid sequence shown in SEQ ID NO: 1.

In one embodiment, the mutation is a substitution, insertion, deletion or addition. In one embodiment, the substitution is a conservative amino acid substitution.

In one embodiment, the collagen is derived from human. In one embodiment, the collagen has a triple-helix structure. In one embodiment, the collagen has a flexible triple-helix structure. In one embodiment, the collagen is in the form of a trimer.

In one embodiment, the collagen has one or more of the following activities or effects: cell adhesion activity, cell migration-promoting activity, transdermal absorption, anti-wrinkle effect, sebum control effect, skin-restoring effect, and skin-soothing effect.

In one aspect, the present application provides a nucleic acid encoding the collagen described herein. In one embodiment, the nucleic acid has the nucleotide sequence shown in SEQ ID NO: 2.

In one aspect, the present application provides a vector comprising the nucleic acid described herein. In one embodiment, the vector comprises a nucleotide encoding a purification tag, a nucleotide encoding a leader, and/or a regulatory element.

In one embodiment, the purification tag is selected from a His tag, GST tag, MBP tag, SUMO tag, or NusA tag.

In one embodiment, the regulatory element is selected from a promoter, terminator, and/or enhancer.

In one aspect, the present application provides a host cell comprising the nucleic acid or vector described herein. In one embodiment, the host cell is a eukaryotic or prokaryotic cell. In one embodiment, the eukaryotic cell is a yeast cell, animal cell, and/or insect cell. In one embodiment, the prokaryotic cell is an *Escherichia coli* cell. In one embodiment, the prokaryotic cell is *E. coli* BL21.

In one aspect, the present application provides a method for producing the collagen described herein, comprising:
(1) culturing the host cell described herein under a suitable condition;
(2) harvesting the host cell and/or medium containing the collagen; and
(3) purifying the fusion protein.

In one embodiment, the method comprises (i) performing crude purification of the collagen using a Ni affinity chromatography column; (ii) adding collagenase for enzymatic cleavage; and/or (iii) performing fine purification of the collagen using an ion exchange column.

In one aspect, the present application provides a composition comprising the collagen described herein, the nucleic acid described herein, the vector described herein and/or the host cell described herein.

In one embodiment, the composition is a pharmaceutical composition, food composition, or cosmetic composition.

In one embodiment, the composition is one or more of the following: biological dressings, human body bionic materials, plastic surgery and beauty materials, organoid culture materials, cardiovascular stent materials, coating materials, tissue injection filler materials, ophthalmic materials, obstetrics and gynecology biomaterials, nerve repair and regeneration materials, liver tissue materials as well as vascular repair and regeneration materials, 3D-printed artificial organ biomaterials, cosmetic raw materials, pharmaceutical excipients, and food additives. In one embodiment, the composition is a solution dosage form, a lyophilized powder dosage form, a gel dosage form, a sponge dosage form or a fiber dosage form.

In one embodiment, the cosmetic composition is a cosmetic composition with anti-wrinkle effect, sebum control effect, skin-restoring effect and/or skin-soothing effect. In one embodiment, the sebum control effect is the sebum control effect on face.

In one embodiment, the composition comprises a pharmaceutically, cosmetically and/or food acceptable carrier.

In one embodiment, the composition is a solid, liquid or gel composition.

In one embodiment, the composition is a composition for oral and/or topical administration. In one embodiment, the topical administration is topical administration to the skin. In one embodiment, the composition is a transdermal composition. In one embodiment, the composition is a kit.

In one embodiment, the composition is a liquid formulation comprising the collagen described herein and a pharmaceutically, cosmetically and/or food acceptable carrier. In one embodiment, the carrier is a buffer, such as a D-PBS buffer or a PBS buffer.

In one aspect, the present application provides a method for promoting cell adhesion or attachment, comprising contacting the cell with the collagen, composition, and/or liquid formulation described herein.

In one aspect, the present application provides a method for promoting cell migration, comprising contacting the cell with the collagen and/or composition described herein.

In another aspect, the present application provides a cosmetic or beauty method, comprising administering the collagen and/or composition described herein to the skin. In one embodiment, the cosmetic or beauty method comprises administering another beauty and/or cosmetic product to the skin.

In one embodiment, the cosmetic or beauty method is a method for delivering anti-wrinkle, sebum control, skin restoration, and/or skin-soothing treatments. In one embodiment, the administration is topical administration. In one embodiment, the administration is transdermal administration.

In yet another aspect, the present application provides the use of the collagen, nucleic acid, vector, and/or host cell described herein in a medical, cosmetic, or beauty product.

In one embodiment, the product is an external use product. In one embodiment, the cosmetic or beauty product is an anti-wrinkle, sebum control, skin-restoring and/or skin-soothing product. In one embodiment, the product is selected from biological dressings, human body bionic materials, plastic surgery and beauty materials, organoid culture materials, cardiovascular stent materials, coating materials, tissue injection filler materials, ophthalmic materials, obstetrics and gynecology biomaterials, nerve repair and regeneration materials, liver tissue materials as well as vascular repair and regeneration materials, or 3D-printed artificial organ biomaterials.

In yet another aspect, the present application provides the use of the collagen, nucleic acid, vector, and/or host cell described herein in the manufacture of a medicament or kit for anti-inflammation or reducing an inflammatory cytokine. In one embodiment, the inflammatory cytokine is TNF-α. In one embodiment, inflammation is caused by an inflammatory cytokine, such as TNF-α.

In yet another aspect, the present application provides a method for reducing an inflammatory cytokine level in a cell, comprising contacting the cell with the collagen, composition, and/or liquid formulation described herein.

The advantages of the present application include:
1) The collagen of the present application is engineered using synthetic biology technologies. Its amino acid sequence is 100% identical to that of the core functional region of human collagen. It is the only available small-molecule protein featuring a 164.88° flexible triple-helix structure.
2) The collagen of the present application is a small-molecule collagen with a molecular weight of approximately 2814.07 Da, capable of penetrating the dermis to reach subcutaneous tissues. Studies demonstrate a skin penetration rate exceeding 85% after 12 h of application to the skin, showcasing exceptional transdermal absorption performance. This performance is significantly superior to that of high-molecular-weight collagens available on the market.
3) The collagen of the present application has a well-defined structure: its triple-helix structure has been successfully resolved by internationally advanced X-ray crystallography, and the relevant data have been deposited in the Protein Data Bank (PDB: 6A0A and 6A0C). This makes it the only commercially available small-molecule collagen with a triple-helix structure.
4) The collagen of the present application exhibits superior bioactivity and hydrophilicity: its cell adhesion activity is significantly higher than that of human collagen, reaching twice that of human type I collagen.
5) The collagen of the present application exerts anti-wrinkle, sebum control, skin-restoring, skin-soothing, and/or anti-inflammatory effects.

### Description of the drawings

Fig. 1 shows the expression vector map.
Fig. 2 shows the electrophoresis detection results during the collagen purification process. Lane 1: marker; Lane 2: purified sample; Lane 3: collagen after buffer exchange; Lane 4: collagen after enzymatic cleavage.
Fig. 3 shows the mass spectrometry detection results of the small-molecule collagen C3T1 of the present application.
Fig. 4 shows the purity detection of the small-molecule collagen C3T1 of the present application.
Fig. 5 shows the cell adhesion assay results of the collagen, with the collagen compared to a control group. * represents P < 0.05, ** P < 0.01, *** P < 0.001. Based on the figure, the data indicate that the present collagen exhibits significantly enhanced cell adhesion activity relative to bovine type I collagen.
Fig. 6 shows fluorescence microscopy observation results.
Fig. 7 shows a bar graph of the effect of the collagen on cell migration.
Fig. 8 shows the actual diffusion percentage of the collagen sample. As can be seen from the figure, the penetration rate of the collagen gradually increases with time and can reach up to 86% at 12 h.
Fig. 9 shows a qualitative analysis of the actual diffusion of the collagen sample. Blue indicates DAPI fluorescence of cell nuclei, and green represents fluorescence of the FITC-labeled sample. As shown in the figure, the sample predominantly remains on the skin surface at 2 h, whereas after 4 h, 8 h, and 12 h, it penetrates through the epidermis into the dermis.
Fig. 10 shows a bar graph illustrating the type I collagen content in each group. Error bars represent the 95% confidence interval. As shown in the figure, treatment with sample concentrations of 25%-100% significantly increased type I collagen protein content (p < 0.05), demonstrating the ability to promote type I collagen synthesis and indicating the sample's pronounced anti-wrinkle effect.
Fig. 11 shows a bar graph of the relative expression amount of filaggrin (FLG) in each group. Error bar: 95% confidence interval.
Fig. 12 shows a bar graph of the relative expression amount of TGM1 in each group. Error bar: 95% confidence interval.
Fig. 13 shows a bar graph of TNF-α content in each group. Error bar: 95% confidence interval.

### Detailed description of the invention

To clarify the objectives, technical solutions, and advantages of the present application, the technical solutions in the examples of this application will be described below in clear and complete detail in conjunction with the accompanying examples. It should be noted that the described examples are merely representative of some, but not all, embodiments of the present application. All other embodiments derived from the examples of this application by those skilled in the art without creative effort shall fall within the protection scope of the present application.

As used herein, a peptide or polypeptide refers to a plurality of amino acid residues linked by peptide bonds. In the application, collagen may also be referred to as collagen peptide or polypeptide.

As used herein, "nucleic acid" refers to a plurality of nucleotides connected via internucleotide linkages, which may include, for example, phosphodiester bonds. The nucleic acid described herein may encompass a polynucleotide encoding the collagen of the present application. To facilitate downstream processing of the collagen, the nucleic acid of the present application may further comprise a nucleotide sequence encoding a purification tag (e.g., His tag, GST tag, MBP tag, SUMO tag, or NusA tag) and, if necessary, a nucleotide sequence encoding a leader peptide.

As used herein, the term "vector" is a nucleic acid delivery vehicle into which a polynucleotide can be inserted. When the vector enables the expression of the protein encoded by the inserted polynucleotide, the vector is referred to as an expression vector. The vector can be introduced into the host cell through transformation, transduction or transfection, allowing the genetic elements it carries to be expressed in the host cell. The vector is well known to those skilled in the art, including but not limited to: plasmid; phagemid; cosmid; artificial chromosome, such as yeast artificial chromosome (YAC), bacterial artificial chromosome (BAC) or P1-derived artificial chromosome (PAC); phage such as lambda phage or M13 phage and animal virus, etc. The vector can contain a variety of expression control elements, including but not limited to promoter sequences, transcription initiation sequences, enhancer sequences, selection elements and reporter genes. In addition, the vector may also contain a replication origin site. The vector may contain the nucleic acid of the present application for ease of introduction into cells for expression. The vector may contain expression control elements, such as promoters, terminators and/or enhancers, operably linked to the nucleic acid.

As used herein, the term "recombinant human collagen" refers to the full-length amino acid sequence encoded by a specific type of human collagen gene prepared by DNA recombinant technology, with a triple-helix structure. As used herein, the term "recombinant humanized collagen" refers to a polypeptide comprising a full-length or partial amino acid sequence derived from a specific human collagen gene, generated via DNA recombinant technology, or a combination containing functional fragments of human collagen.

As used herein, the term "host cell" is a cell into which nucleic acid molecules have been introduced by molecular biology techniques. These techniques include transfection with viral vectors, transformation with plasmid vectors, and accelerated introduction of naked DNA via electroporation, lipofection, and particle guns. The host cell can be a eukaryotic or prokaryotic cell. The eukaryotic cell is, for example, a yeast cell, animal cell and/or insect cell. The prokaryotic cell can be an *E. coli* cell.

As used herein, the degree of relatedness between two amino acid sequences or between two nucleotide sequences is described by the parameter "sequence identity". For the purposes of the present application, the sequence identity between two amino acid sequences is determined by the Needleman-Wunsch algorithm (Needleman and Wunsch, 1970, J. Mol. Biol. 48: 443-453) as implemented in the Needle program of the EMBOSS software package (EMBOSS: The European Molecular Biology Open Software Suite, Rice et al., 2000, Trends Genet. 16: 276-277) (preferably version 5.0.0 or later). The parameters used are a gap opening penalty of 10, a gap extension penalty of 0.5, and the EBLOSUM62 (EMBOSS version of BLOSUM62) substitution matrix. The output labeled "longest identity" from the Needle (obtained by the non-simplified option) is used as the percentage identity and calculated as follows:
(identical residues × 100)/(alignment length - total number of gaps in the alignment)

For the purposes of the present application, the sequence identity between two deoxynucleotide sequences is determined by the Needleman-Wunsch algorithm (Needleman and Wunsch, 1970, ibid.) as implemented in the Needle program of the EMBOSS software package (EMBOSS: The European Molecular Biology Open Software Suite, Rice et al., 2000, ibid.) (preferably version 5.0.0 or later). The parameters used are a gap opening penalty of 10, a gap extension penalty of 0.5, and the EDNAFULL (EMBOSS version of NCBI NUC4.4) substitution matrix. The output labeled "longest identity" from the Needle (obtained by the non-simplified option) is used as the percentage identity and calculated as follows:
(identical deoxynucleotides × 100)/(alignment length - total number of gaps in the alignment)

In the context of the present application, a conservative amino acid substitution or conservative substitution can be defined by a substitution within the amino acid categories reflected in one or more of the following tables:
Conservative Categories of Amino Acid Residues:

| | |
|---|---|
| Acidic residues | D and E |
| Basic residues | K, R and H |
| Hydrophilic uncharged residues | S, T, N and Q |
| Aliphatic uncharged residues | G, A, V, L and H |
| Nonpolar uncharged residues | C, M and P |
| Aromatic residues | F, Y and W |

Alternative physical and functional classifications of amino acid residues:

| | |
|---|---|
| Alcohol-containing residues | S and T |
| Aliphatic residues | I, L, V and M |
| Cycloalkenyl-related residues | F, H, W and Y |
| Hydrophobic residues | A, C, F, G, H, I, L, M, R, T, V, W and Y |
| Negatively charged residues | D and E |
| Polar residues | C, D, E, H, K, N, Q, R, S and T |
| Positively charged residues | H, K and R |
| Small residues | A, C, D, G, N, P, S, T and V |
| Very small residues | A, G and S |
| Residues involved in turn formation | A, C, D, E, G, H, K, N, Q, R, S, P and T |
| Flexible residues | Q, T, K, S, G, P, D, E and R |

### Collagen

In the application, the collagen of the present application may have certain mutations. For example, the amino acid sequence of one or more of these portions may have a substitution, deletion, addition or insertion of an amino acid residue. That is, variants can be used in the present application as long as the variant retains the activity of promoting cell adhesion and/or proliferation. Specifically, the variant can have a certain percentage sequence identity to the specified sequence, such as 80%, 85%, 90%, 91%, 92%, 93%, 94%, 95%, 96%, 97%, 98%, or 99% identity. The specified sequence can be any sequence of the present application, such as SEQ ID NO: 1, but it is preferred that these variants retain the function of the collagen of the present application. The collagen of the present application can have good transdermal performance and can directly reach the dermis. The polypeptide of the present application can be used for one or more of the following functions: anti-wrinkle effect, sebum control effect, skin-restoring effect, skin-soothing effect and anti-inflammatory effect.

The collagen of the present application can be prepared by any suitable method, for example, it can be prepared by synthesis. Preferably, the collagen of the present application can be prepared recombinantly.

The collagen of the present application can have a triple-helix structure region. For example, the collagen has a flexible triple-helix structure region. The collagen of the present application can form a triple-helix structure. Upon characterization, the small-molecule collagen C3T1 of the present application can form a 164.88° triple-helix structure.

### Composition

The collagen of the present application can be prepared into a composition. The composition may include the collagen, nucleic acid, vector and/or host cell described herein. The composition may also include a pharmaceutical, cosmetic and/or food acceptable carrier or solvent. The composition may be a pharmaceutical composition, a food composition or a cosmetic composition for the purpose of medicament, food and/or cosmetics. For example, the composition is one or more of the following: biological dressings, human body bionic materials, plastic surgery and beauty materials, organoid culture materials, cardiovascular stent materials, coating materials, tissue injection filler materials, ophthalmic materials, obstetrics and gynecology biomaterials, nerve repair and regeneration materials, liver tissue materials as well as vascular repair and regeneration materials, 3D-printed artificial organ biomaterials, cosmetic raw materials, pharmaceutical excipients, and food additives.

The cosmetic composition may be a cosmetic composition with anti-wrinkle effect, sebum control effect, skin-restoring effect and/or skin-soothing effect. The part to which the cosmetic composition is applied is not particularly limited, and may be the face, hands, legs, trunk, etc. For example, the sebum control effect is the sebum control effect on face.

The form of the composition is not particularly limited, as long as the intended function can be achieved. For example, the composition is a solid, liquid or gel composition.

The composition can be applied in any suitable manner, such as a composition for oral and/or topical administration. The topical administration can be topical administration to the skin. Due to the transdermal properties of the composition, the composition can be prepared into a transdermal product or a transdermal cosmetic. The composition can also be prepared into a kit. The kit can contain additional ingredients, such as auxiliary ingredients such as a buffer, and instructions for use. In particular, the composition can be formulated into a suitable formulation, such as a liquid formulation. The formulation can contain a buffer, such as a D-PBS buffer or PBS buffer.

### Method

The present application provides a method for promoting cell adhesion or attachment, comprising contacting the cell with the collagen, composition, and/or liquid formulation described herein. The method of the present application can be performed in vitro to increase the adhesion of cells to a culture vessel. Alternatively, the method of the present application can also be performed in vivo.

The present application further provides a method for promoting cell migration, comprising contacting the cell with the collagen and/or composition. The method of the present application can be performed in vitro or in vivo.

The present application further provides a cosmetic or beauty method, comprising administering the collagen and/or composition described herein to the skin. The administration to the skin can be topical administration, such as administration to the skin area that needs anti-wrinkle, sebum control, skin restoration and/or skin-soothing treatments. The method may further include applying another beauty and/or cosmetic product to the skin. The types of other beauty and/or cosmetic product are not particularly limited, and can be other anti-wrinkle, sebum control, skin-restoring and/or skin-soothing cosmetics, such as collagen. These cosmetic or beauty methods can be methods for delivering anti-wrinkle, sebum control, skin restoration, and/or skin-soothing treatments. Due to the excellent transdermal penetration of the collagen described herein, it can reach the inner layers of the skin through the epidermis after topical application.

The present application further provides a method for administering the collagen or composition described herein to a subject in need thereof. The method can be used to treat or prevent conditions related to collagen deficiency, or can be used for delivering anti-wrinkle, sebum control, skin restoration and/or skin-soothing treatments in a subject. The administration can be oral administration.

The present application further provides a method for reducing the level of an inflammatory cytokine in a cell, comprising contacting the cells with the collagen, composition, and/or liquid formulation described herein. The type of the inflammatory cytokine is not particularly limited, but preferably TNF-α.

### Use

The present application further provides the use of the collagen, nucleic acid, vector, and/or host cell in a medical, cosmetic, or beauty product or the use for delivering anti-wrinkle, sebum control, skin restoration, and/or skin-soothing treatments. The product can be external use products, such as external cosmetic products or beauty products. These cosmetic or beauty products can be anti-wrinkle, sebum control, skin-restoring and/or skin-soothing products. For example, the product can be selected from biological dressings, human body bionic materials, plastic surgery and beauty materials, organoid culture materials, cardiovascular stent materials, coating materials, tissue injection filler materials, ophthalmic materials, obstetrics and gynecology biomaterials, nerve repair and regeneration materials, liver tissue materials as well as vascular repair and regeneration materials, or 3D-printed artificial organ biomaterials.

The present application further provides the use of the collagen, nucleic acid, vector, and/or host cell described herein in the manufacture of a medicament or kit for anti-inflammation or reducing an inflammatory cytokine such as TNF-α.

### Examples

The following examples are provided to illustrate the present application. Those skilled in the art should understand that the examples are merely illustrative and not restrictive. The present application is limited only by the scope of the appended claims.

### Example 1: Construction and expression of small-molecule collagen C3T1

### 1. Construction of E. coli genetic engineering strain

The amino acid sequence corresponding to the small-molecule collagen C3T1 is: GERGAPGFRGPAGPNGIPGEKGPAGERGAP (SEQ ID NO: 1). The corresponding nucleotide sequence of the amino acid sequence is: GGAGAAAGGGGGGCGCCAGGCTTCCGCGGTCCGGCGGGTCCGAACGG CATCCCGGGTGAGAAGGGTCCGGCTGGCGAACGTGGCGCACCG (SEQ ID NO: 2). The nucleotide sequence of the small-molecule collagen C3T1 was cloned into an expression vector, and then the expression vector was transferred into an *E. coli* expression strain to screen and obtain the *E. coli* genetic engineering strain.

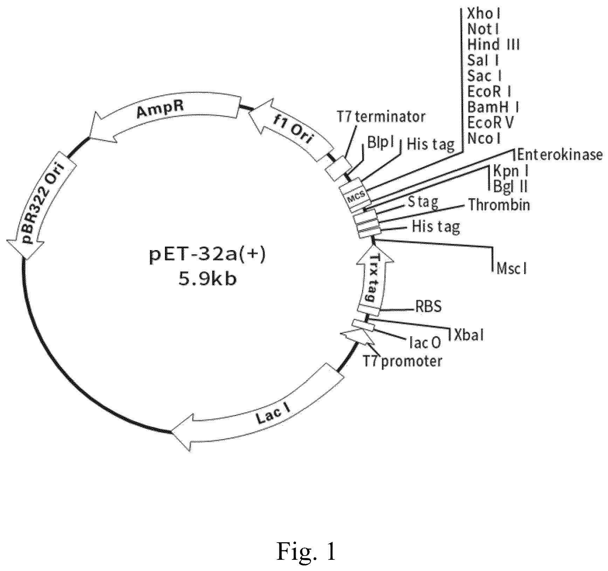

Specifically, based on the amino acid sequence of C3T1, the gene SEQ ID NO: 2 was selected through codon optimization for *E. coli* preference. The nucleotide sequence of SEQ ID NO: 2 was synthesized, and the C3T1 gene fragment was inserted into the pET-32a expression vector (Beijing Shengyuan Kemeng Biotechnology Co., Ltd.) via the Kpn I (New England Biolabs (NEB), Cat. No. R0136L) and Xho I (New England Biolabs (NEB), Cat. No. R0146L) restriction sites, generating the pET-32a-C3T1 expression vector (Fig. 1). Subsequently, the expression vector was transformed into *E. coli* BL21 (DE3), and positive genetic engineering strains were screened. All above procedures were commissioned to Beijing Shengyuan Kemeng Biotechnology Co., Ltd.

### 2. Fermentation and culture of E. coli genetic engineering strain

a. A single colony of the selected *E. coli* genetic engineering strain was picked and inoculated into 5 mL of LB medium at 35-38°C and cultured overnight at 37°C. b. The culture was inoculated into a medium at a 1:100 dilution for scale-up culture. After growing at 37°C for 7 h, protein expression was induced by adding 0.5 mM IPTG. The culture was then cooled to 16°C and incubated overnight for induction. The expressed protein was C3T1. The bacteria were collected by centrifugation. c. The collected bacteria were resuspended in equilibration buffer (200 mM NaCl, 25 mM Tris, 20 mM imidazole, pH 8.0). The bacterial solution was cooled to ≤15°C, homogenized twice using high-pressure homogenization, and the bacterial lysate was collected after completion of the homogenization. The homogenized bacterial solution was divided into centrifuge tubes, centrifuged at 17000 rpm and 4°C for 30 min. The supernatant was collected, and both the supernatant and precipitate were taken for electrophoresis detection.

### 3. Purification of the small-molecule collagen C3T1

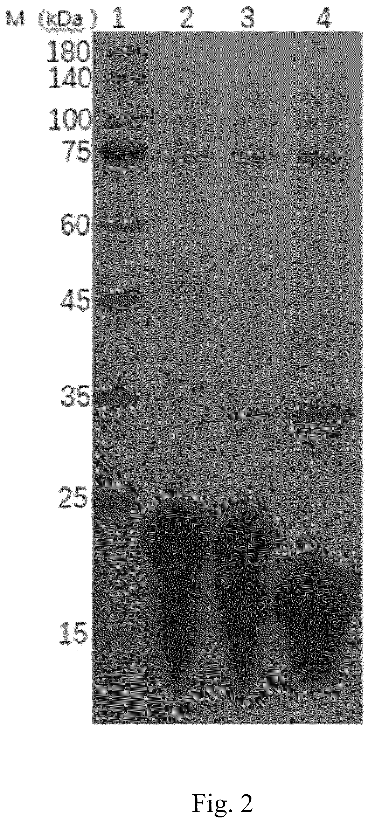

a. The column material (Ni6FF, Cytiva) was washed with water for 5 column volumes (CV). b. The column material was equilibrated with an equilibration buffer (200 mM NaCl, 25 mM Tris, 20 mM imidazole) for 5 CV. c. Loading: The centrifuged supernatant was loaded onto the column until the liquid had completely flowed through. d. Washing off impurities: 25 mL of wash buffer (200 mM NaCl, 25 mM Tris, 20 mM imidazole) was added and passed through the column until the liquid had completely flowed through. e. Collection of the target protein: 25 mL of elution buffer (200 mM NaCl, 25 mM Tris, 250 mM imidazole) was applied, and the flow-through was collected to obtain the target polypeptide Trx-His-C3T1. f. Buffer exchange: The collected target protein was subjected to buffer exchange into PBS (300 mM NaCl, 50 mM sodium dihydrogen phosphate, pH 7.3) using a G-25 column material (Cytiva). If the removal of the Trx-His tag was required from the target protein, an appropriate amount of collagenase was added and the mixture was incubated at 16°C for 2 h, yielding the Trx-His-tag-removed small-molecule collagen C3T1. The electrophoresis detection results are shown in Fig. 2 (Lane 1: marker; Lane 2: collected target protein; Lane 3: target protein after buffer exchange to PBS; Lane 4: sample after enzymatic cleavage).

### 4. Mass spectrometry detection of the small-molecule collagen C3T1

The theoretical molecular weight of the small-molecule collagen C3T1 is 2814.07 Da, which cannot be detected by conventional SDS-PAGE and requires identification via mass spectrometry. A Biozen^{™} 1.8 µm dSEC-2, 200 Å, 150 × 4.6 mm LC column was selected, and the enzymatically cleaved sample was analyzed using a LC-MS device with the molecular weight detection mass spectrometry (MS) method and liquid chromatography (LC) method.

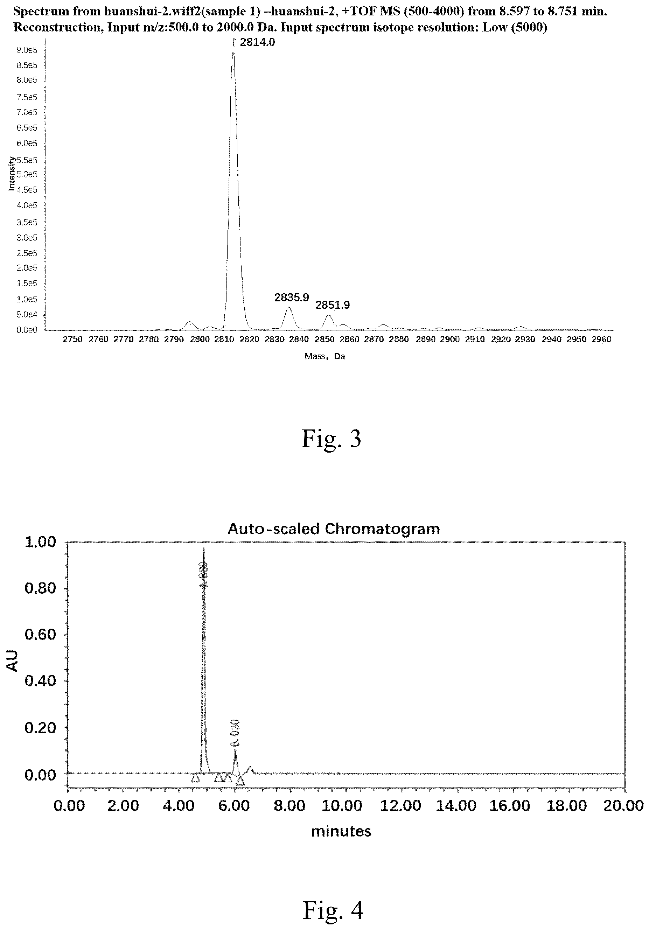

As shown in Fig. 3, according to identification by the mass spectrometry method, the actual molecular weight of the small-molecule collagen C3T1 of the present application is 2814Da, which is consistent with the theoretical molecular weight.

### 5. Purity detection of the small-molecule collagen C3T1

The testing method followed the Chinese Pharmacopoeia (2020 Edition), Part IV, General Rule 0512 (High-Performance Liquid Chromatography). The protein test sample was analyzed using molecular exclusion chromatography, and the purity percentage of the main peak was calculated using the peak area normalization method.

The blank control and collagen C3T1 samples were loaded into the sample tray for analysis. The experimental parameters were set as follows:

| | | | | |
|---|---|---|---|---|
| Chromatographic column | Acquity UPLC Protein BEH SEC, 2.7 µm, 4.6 × 150 mm | | | |
| Mobile phase | Phase A | | 0.1% TFA aqueous solution | |
| | Phase B | | 0.1% TFA acetonitrile solution | |
| Elution program | Time (min) | A% | | B% |
| | 0 | 65 | | 35 |
| | 20 | 65 | | 35 |
| Flow rate | 0.3 ml/min | | | |
| Column temperature | 40° C | | | |
| Detection wavelength | 214 nm | | | |
| Injection volume | 5µl | | | |

The test results were as follows:

**Table 1**

| Chromatographic peak | Sample | Retention Time (min) | Area (microvolt * second) | % Area |
|---|---|---|---|---|
| 1 | Protein | 4.889 | 5582783 | 87.70 |
| 2 | -- | 6.030 | 783115 | 12.30 |

Fig. 4 shows the purity detection of the small-molecule collagen C3T1 of the present application. The purity of the small-molecule collagen C3T1 was as high as 87.70%.

### Example 2: Cell adhesion activity test of the small-molecule collagen C3T1

The cell adhesion activity test was performed by referring to the Chinese Pharmaceutical Industry Standard YY/T 1849-2022 "Recombinant collagen."

The specific implementation method was as follows:
The sample was dissolved in D-PBS, filtered through a 0.22 µm membrane, and diluted to the working concentration for use. The positive control, bovine type I collagen (Hebei Kaolisen Biotechnology Co., Ltd.; Cat. No. CSC113A) was diluted to 1 mg/mL with D-PBS for later use. The negative control was D-PBS buffer.

Different concentrations (0.5, 1, or 2 mg/mL) of collagen C3T1, along with the positive and negative controls were added to the wells of an ELISA plate with 100 µL per well, five replicate wells for each group, and incubated overnight at 4°C.

Each well was added with 10⁵ well-cultured 3T3/NIH cells suspended in D-PBS and incubated at 37°C for 120 minutes. Each well was washed with D-PBS solution for 3 times.

The absorbance at OD450 nm was measured with CCK8 detection kit (Vazyme; Cat. No. A311-01-AA) according to the instructions of the product. The cell adhesion degree was calculated according to the following formula: cell adhesion rate = (test well - blank well) × 100% / (positive well - blank well). The cell adhesion rate can reflect the cell-adhesive capacity of collagen. The higher the cell adhesion ability, the more effectively an optimal external environment can be provided to the cells within a short timeframe, facilitating their adhesion to the plate surface.

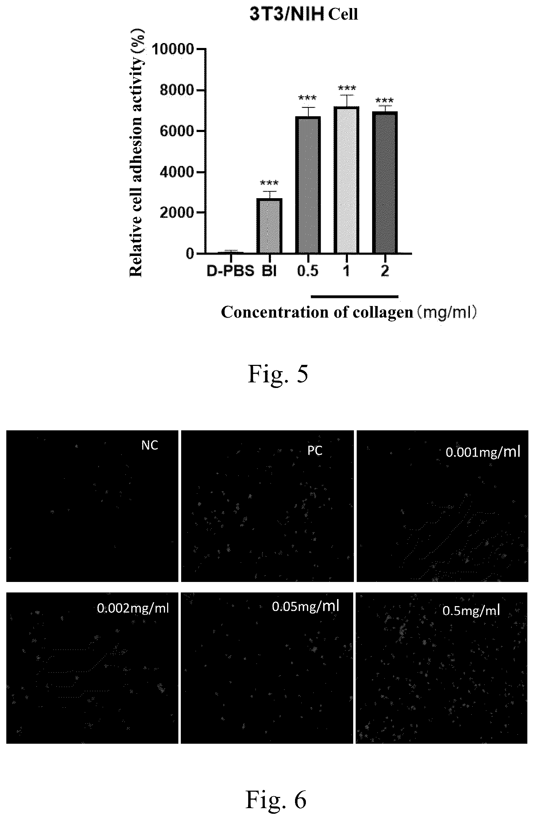

See Fig. 5 for the results. As can be seen from Fig. 5, the collagen C3T1 of the present application has better adhesion activity than bovine type I collagen.

### Example 3: Cell migration-promoting activity test of the small-molecule collagen C3T1

The negative control (NC) was a medium containing 0.4% serum (ECM: 500 mL basal medium + 25 mL FBS serum + 5 mL ECGS endothelial cell growth supplement + 5 mL P/S penicillin/streptomycin solution), the positive control (PC) was EGF, and the test sample was collagen C3T1. The cultured cells were HUVEC cells. The collagen was dissolved in ECM medium containing 0.4% serum to reach the working concentration, sterilized by filtration through a 0.22 µm membrane, and then serially diluted using the half-dilution method. The final concentration of collagen C3T1 in the medium was 0.001, 0.002, 0.05 and 0.5 mg/mL. EGF was dissolved in ECM medium containing 0.4% serum to reach a final concentration of 10 ng/ml and filtered for sterilization. The cells were digested. After terminating digestion, the culture medium was discarded by centrifugation, and the cells were washed 1-2 times with PBS. The cells were resuspended in serum-free medium, and the cell density was adjusted to 1×10⁶ cells/mL. A two-chamber system (Nest, cell chamber culture plate) was used. The lower chamber was filled with 500 µL of the sample, and 200 µL of the cell suspension was added to the upper chamber, followed by incubation for 12-48 h. The upper chamber was carefully removed with tweezers, the liquid was aspirated, and the chamber was transferred to a well pre-filled with approximately 800 µL of methanol. Cells were fixed at room temperature for 30 minutes and then washed twice with PBS. 100 µL of DAPI was added to the upper chamber. After staining for 15 minutes, non-migrated cells on the upper layer were gently removed with a cotton swab, and the chamber was washed three times with PBS. The cells were observed and photographed under a fluorescence microscope, and ImageJ was used for counting. The calculation formula was as follows: relative cell migration rate = average count of the experimental group / average count of the negative control group × 100%.

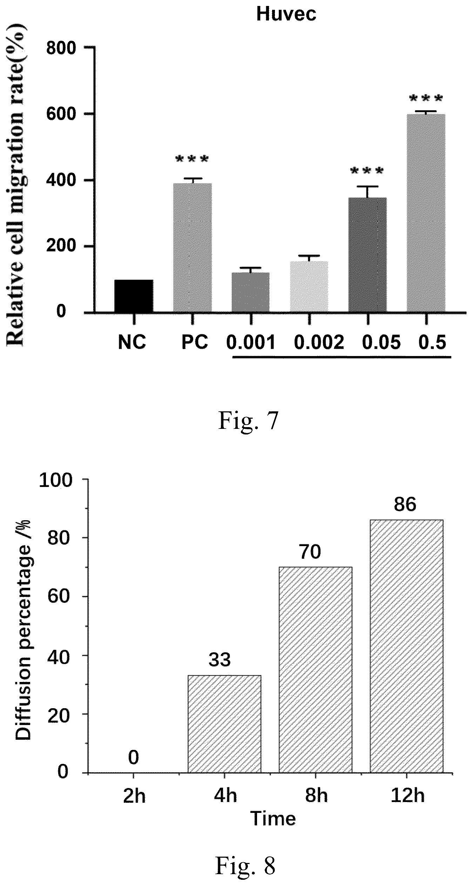

Fig. 6 shows the fluorescence microscopy observation results of different test groups. Fig. 7 shows the relative cell migration rate (%) of different test groups.

According to Fig. 7, the positive control group can promote cell migration compared with the negative control group, and the difference is statistically significant. As the concentration of collagen C3T1 increased, its effect on promoting cell migration gradually strengthened. Cell migration activity is an indicator of the biological activity of collagen. The higher the migration rate, the higher the biological activity of collagen. Compared with EGF, collagen C3T1 has higher cell migration activity.

### Example 4: Transdermal absorption test of the small-molecule collagen C3T1

The transdermal absorption test of the small-molecule collagen C3T1 refers to the standard method, GB/T 27818-2011 "In Vitro Test Method for Skin Absorption of Chemicals."

The specific implementation method was as follows:
Quantitative determination steps for transdermal absorption: the receiving chamber was filled with receiving solution (physiological saline), and the matching magnetic stir bar was placed in the receiving chamber. Suckling pig skin (with the test product placed on the surface of the suckling pig skin) was fixed between the diffusion chamber and receiving chamber of the Franz cell diffusion apparatus. An additional 1 mL of receiving solution was added to the sampling tube, so that the dermis of the suckling pig skin was in close contact with the receiving solution. The Franz cell diffusion cell was secured in the transdermal absorption diffusion device. The magnetic stirrer was turned on and set to 300 rpm, and the water bath interlayer was kept bubble-free. After the temperature stabilized, 400 µL of the sample was drawn onto the suckling pig skin surface. 1 ml of the receiving solution was extracted at 2, 4, and 8 h respectively, placed in a 2 ml EP tube, and then an appropriate amount of physiological saline solution was added to the receiving chamber, and the sample was collected at 12 h. Treatment of the unpenetrated part on the skin: after the penetration for 12h (sample), the surface of the suckling pig skin was cleaned with physiological saline and the volume was adjusted to 2 ml. Treatment of the residual part in the skin: after the penetration for 12 h (sample), the suckling pig skin was cut into pieces and placed in an EP tube, and the volume was adjusted to 2 ml with physiological saline, and ultrasonicated for 30 minutes. Samples were collected at each time point and analyzed for collagen C3T1 content via the Folin phenol method (the test product was applied to the surface of suckling pig skin).

Qualitative determination steps for transdermal absorption: the receiving chamber was filled with the receiving solution, and the matching magnetic stir bar was placed in the receiving chamber. Suckling pig skin was fixed between the diffusion chamber and receiving chamber of the Franz cell diffusion cell. An additional 1 mL of the receiving solution was added to the sampling tube, so that the dermis of the suckling pig skin was in close contact with the receiving solution. The Franz cell diffusion cell was secured in the transdermal absorption diffusion device. The magnetic stirrer was turned on and set to 300 rpm, and the water bath interlayer was kept bubble-free. After the temperature stabilized, 400 µL of the collagen C3T1 (pre-labeled with FITC (fluorescein isothiocyanate)) was drawn onto the suckling pig skin surface. Qualitative analysis: Suckling pig skin samples from each group were collected for frozen sectioning, followed by DAPI counterstaining and imaging under a fluorescence microscope. Three parallel experiments were performed according to the above method. Cumulative penetration calculation formula: Q=Cn×V+ΣCi×V₀ (i=1···n-1)
in which Q: cumulative penetration amount
V: receiving solution volume in the receiving chamber
V₀: volume of each sampling
Ci: the target concentration of the receiving solution at the 1st to (n-1)th sampling time points
Cn: target concentration measured at the nth sampling point.

Diffusion percentage was calculated according to the formula: P=Q/P₀×100%
in which: P: diffusion percentage
Q: cumulative penetration amount at each timepoint in the receiving chamber
P₀: initial loading amount in the diffusion chamber.

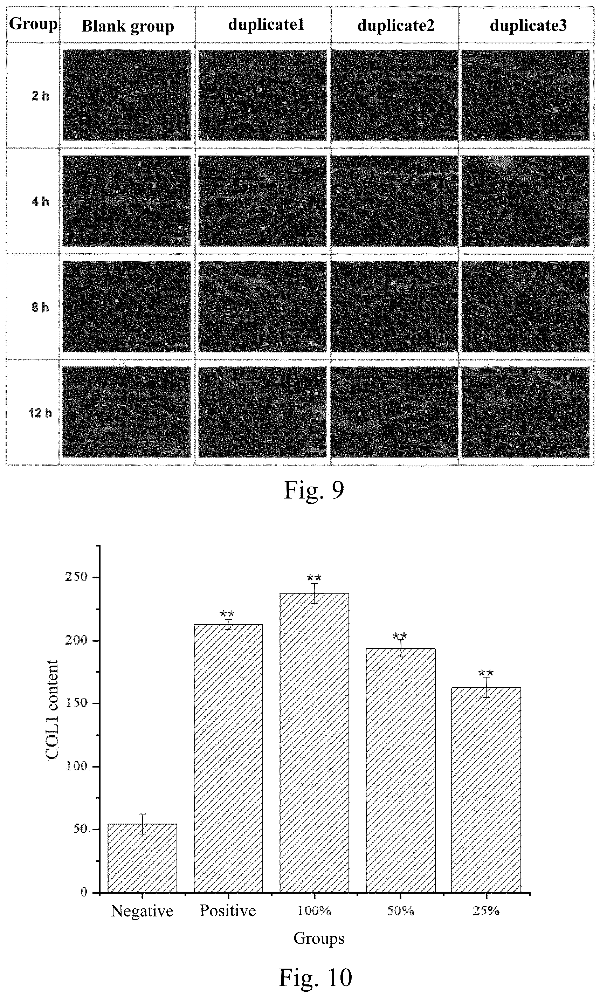

The results are shown in Fig. 8. As depicted in Fig. 8, the penetration rate of collagen C3T1 exhibited a gradual increase over time, reaching up to 86% at 12 h. Fig. 9 illustrates the fluorescence microscopy qualitative analysis results. As shown in Fig. 9, the sample remained predominantly on the skin surface at 2 h, whereas after 4, 8, and 12 h, the sample penetrated through the skin epidermis into the dermis.

In summary, after subtracting the blank group data from the collagen test group results. At 2 h, the sample remained primarily on the skin surface. At 4 h, the collagen penetration rate of collagen C3T1 was 33%, and FITC-labeled collagen C3T1 penetrated through the epidermal layer into the dermis. At 8 h, the collagen penetration rate reached 70%, and FITC-labeled collagen C3T1 penetrated through the epidermal layer into the dermis. At 12 h, the collagen penetration rate reached 86%, and FITC-labeled collagen C3T1 penetrated through the epidermal layer into the dermis. These findings indicate that collagen C3T1 is a genuine small-molecule collagen with superior transdermal absorption capacity, capable of directly reaching the dermis to facilitate direct collagen replenishment.

### Example 5: Cytotoxicity test of the small-molecule collagen C3T1

Human fibroblasts (purchased from Guangdong Boxi Biotechnology Co., Ltd.) were seeded in a 96-well plate at a density of 1×10⁴ cells/well and incubated overnight in an incubator (37°C, 5% CO₂). When the cell confluency in the 96-well plate reached 40%-60%, the sample was administered. The zero-adjustment group was cell-free wells with only 200 µL of FbGrowth culture medium (supplier: Guangdong Boxi Biotechnology Co., Ltd.; Cat. No.: PY3091) added to each well. The solvent control group was the cell-inoculated wells which received 200 µL of FbGrowth culture medium in each well. The positive control group was cell-inoculated wells which received 200 µL of FbGrowth culture medium containing 10% DMSO (manufacturer: MACKLIN; Cat. No.: D806645) in each well. The sample group was cell-inoculated wells which received 200 µL of FbGrowth culture medium containing the corresponding concentration of collagen C3T1 in each cell-inoculated well. After sample addition, the 96-well plate was placed in an incubator (37°C, 5% CO₂) for incubation.

After 24 h of cell incubation, the supernatant was removed, and MTT working solution (supplier: YuanYe Bio-Technology Co., Ltd.; Cat. No.: S19063; 0.5 mg/mL) was added. The plate was incubated at 37°C for 4 h protected from light. The supernatant was then removed, and 150 µL of DMSO was added to each well. Absorbance (OD) was measured at 490 nm. Relative cell viability was calculated according to the formula: relative cell viability (%) = (sample well OD - zero-adjustment well OD) ÷ (solvent control well OD - zero-adjustment well OD) × 100%

Table 2 shows the cytotoxicity results.

**Table 2: Cytotoxicity results**

| | | | | | | | | | |
|---|---|---|---|---|---|---|---|---|---|
| Sample concentration (%) | Zero-adjustment well | 100 | 50 | 25 | 12.5 | 6.25 | 3.125 | 1.5625 | 0.78125 |
| Relative survival rate% | / | 98.27 | 100.46 | 100.59 | 101.09 | 101.59 | 102.60 | 101.96 | 99.86 |
| Standard deviation | / | 0.01 | 0.01 | 0.01 | 0.01 | 0.01 | 0.00 | 0.01 | 0.01 |

Table 2 indicates that collagen C3T1 exhibits lower cytotoxicity across the tested concentration gradient, suggesting reliable experimental results.

### Example 6: Determination of the anti-wrinkle effect of the small-molecule collagen C3T1 in vitro

Collagen is the most important component of the extracellular matrix, which can make the skin firmer and more elastic. A reduction in collagen leads to visible wrinkles. Human dermal fibroblasts can synthesize type I procollagen in vivo and secrete it extracellularly, where the type I procollagen polymerizes to form collagen fibers. Therefore, human dermal fibroblasts can serve as a cellular model to study the promotion of type I collagen synthesis by cosmetics, evaluating whether the test substance has the effect of promoting collagen synthesis and thereby evaluating the anti-wrinkle effect of cosmetics.

To determine the anti-wrinkle effect of collagen in vitro, the ability to promote type I collagen synthesis of fibroblasts in vitro was used to illustrate its anti-wrinkle effect.

The specific implementation was as follows:

### Cell culture:

Human fibroblasts in the logarithmic growth phase were collected and inoculated into 96-well plates at a cell density of 1×10⁴/well, cultured in a 37°C, 5% CO₂ incubator for 24 h. Samples were added when the cell confluency in the 96-well plate reached 40%-60%. The blank control group was FbGrowth culture medium added to the wells inoculated with cells. The positive control group was TGFβ1 (supplier: Absin Bioscience Inc.; Cat. No.: ABS04204) added to the wells inoculated with cells (10 µg TGFβ1 was weighed and dissolved in 200 µL 10 mM citric acid solution as a stock solution; 2 µL TGFβ1 stock solution was aspirated and dissolved in 998 µL FbGrowth culture medium as a positive control working solution). The sample group was FbGrowth culture medium with various concentrations of collagen C3T1 added to the wells inoculated with cells. After the sample was added, the 96-well plate was placed in an incubator (37°C, 5% CO₂) and cultured for 24 h.

After the culture was completed, the cells were collected in EP tubes and type I collagen was detected using a type I collagen ELISA kit (supplier: ELK Biotechnology co., Ltd.; Cat. No.: ELK2377; see the kit instructions for detailed operations).

Test results: As can be seen from Fig. 10, when the sample concentration was 25%-100% (concentration was 0.075-0.3 µg/mL), the protein content of type I collagen can be significantly increased (p < 0.05), demonstrating the ability to promote type I collagen synthesis, indicating that the collagen C3T1 sample has an obvious anti-wrinkle effect.

### Example 7: In vitro facial sebum control effect of the small-molecule collagen C3T1

Cell culture: KC human epidermal cells (purchased from Guangdong Boxi Biotechnology Co., Ltd.) in the logarithmic growth phase were collected and inoculated into 96-well plates at a cell density of 1×10⁴ cells/well. KC human epidermal cells were cultured in a 37°C, 5% CO₂ incubator for 24 h, and the sample was added when the cell confluency in the 96-well plate reached 40%-60%. The blank control group was KcGrowth culture medium (supplier: Guangdong Boxi Biotechnology Co., Ltd.; Cat. No.: PY3011) added to the wells inoculated with cells. The negative control group was KcGrowth culture medium with 0.2% SLS (supplier: Solarbio Science &Technology Co., Ltd.; Cat. No.: S8100) added to the wells inoculated with cells. The positive control group was KcGrowth culture medium with 0.2% SLS and WY14643 (supplier: Beyotime; Cat. No.: SD7189-25mg) added to the wells inoculated with cells. The sample group was KcGrowth culture medium with 0.2% SLS and various concentrations of collagen C3T1 added to the wells inoculated with cells. After the sample was added, the 96-well plate was placed in an incubator (37°C, 5% CO₂) and cultured for 24 h.

Detection: After the culture was completed, the cells were collected in EP tubes, the supernatant was taken, and human filaggrin was detected using a human filaggrin ELISA kit (supplier: ELK Biotechnology co., Ltd.; Cat. No.: ELK3513; see the kit instructions for detailed operations).

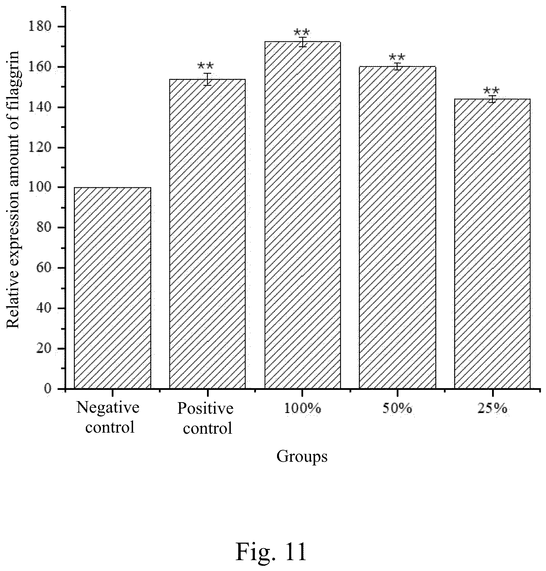

The experimental results are shown in Fig. 11. When the concentration of the test sample was 25%-100% (concentration was 0.075 to 0.3 µg/mL), it could promote the increase of the expression amount of human keratinocyte filaggrin (FLG) in a dose-dependent manner, which was significantly different from the negative control (P<0.05); the test sample collagen C3T1 had a sebum control effect.

### Example 8: In vitro restoration effect test of the small-molecule collagen C3T1

Cell culture: KC human epidermal cells (purchased from Guangdong Boxi Biotechnology Co., Ltd.) in the logarithmic growth phase were collected and inoculated into 96-well plates at a cell density of 1×10⁴ cells/well. KC human epidermal cells were cultured in a 37°C, 5% CO₂ incubator for 24 h. When the cell confluency in the 96-well plate reached 40%-60%, the sample was added. The blank control group was KcGrowth culture medium in the wells inoculated with cells. The negative control group was KcGrowth culture medium with 0.2% SLS added to the wells inoculated with cells. The positive control group was KcGrowth culture medium with 0.2% SLS and WY14643 added to the wells inoculated with cells. The sample group was KcGrowth culture medium with 0.2% SLS and various concentrations of collagen C3T1 added to the wells inoculated with cells. After the sample was added, the 96-well plate was placed in an incubator (37°C, 5% CO₂) and cultured for 24 h.

Detection: After the culture was completed, the cells were collected in EP tubes, the supernatant was taken for TGM1 detection, and human TGM1 was detected using a human TGM1 ELISA kit (supplier: ELK Biotechnology co., Ltd.; Cat. No.: ELK2193; see the kit instructions for detailed operations).

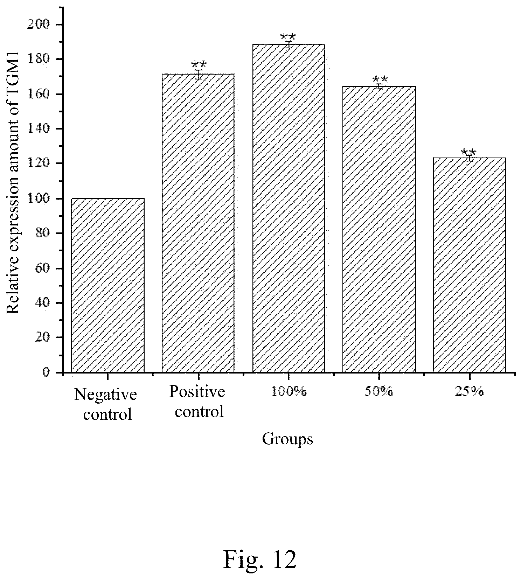

The experimental results are shown in Fig. 12. When the concentration of the test sample was 25%-100% (concentration was 0.075-0.3 µg/mL), it could promote the increase of the expression amount of TGM1 in human keratinocytes in a dose-dependent manner; the test sample collagen C3T1 had a restoration effect.

### Example 9: In vitro soothing effect test of the small-molecule collagen C3T1

Cell culture: Mouse macrophage RAW264.7 cells (purchased from Guangdong Boxi Biotechnology Co., Ltd.) in the logarithmic growth phase were collected and inoculated into 96-well plates at a cell density of 1×10⁴ cells/well. Mouse macrophage RAW264.7 cells were cultured in a 37°C, 5% CO₂ incubator for 24 h. When the cell confluency reached 40%-60%, the sample was added. The blank control group was cell medium (high-glucose DMEM culture medium (10% fetal bovine serum)) (supplier: Biosharp; Cat. No.: BL301A), 200 µL per well. The negative control group was cell medium containing LPS (1 µg/mL; supplier: Absin Bioscience Inc.; Cat. No.: ABS47014848), 200 µL per well. The positive control group was medium containing positive control (10 mg dexamethasone (supplier: Solarbio Science &Technology Co., Ltd.; Cat. No.: D8040) dissolved in 1 mL DMSO as 10 mg/mL stock solution; stock solution diluted 1:100 in high-glucose DMEM containing LPS to 100 µg/mL working solution) and LPS, 200 µL per well. The sample group was medium containing specified concentrations of collagen C3T1 and LPS, 200 µL per well. After sample addition, the plate was incubated (37°C, 5% CO₂) for 24 h.

Detection: After culture completion, cell culture medium was collected. Supernatant was obtained by centrifugation for TNF-α detection using an TNF-α inflammatory factor ELISA kit (supplier: ELK Biotechnology co., Ltd.; Cat. No.: ELK1190; see the kit instructions for the specific detection steps).

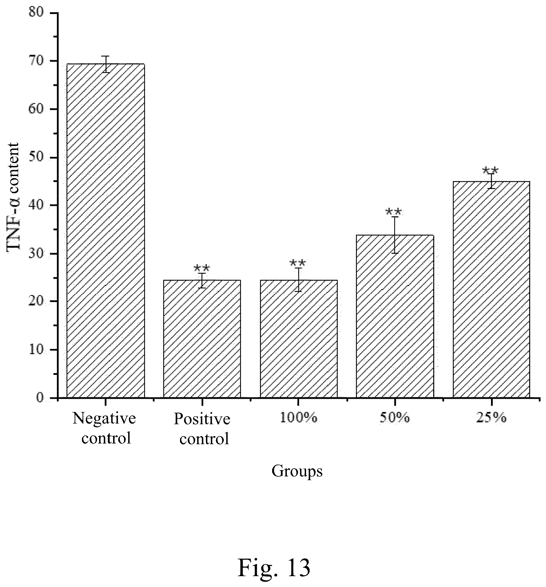

The experimental results are shown in Fig. 13. When the concentration of collagen C3T1 was 25%-100% (concentration was 0.075-0.3 µg/mL), the TNF-α content of each group was reduced compared with the negative control, and there was a significant difference (P<0.05), demonstrating the effect of inhibiting TNF-α content and indicating soothing or anti-inflammatory effects.

The above examples are preferred embodiments of the present application, but the embodiments of the present application are not limited to the above embodiments. Any other changes, modifications, substitutions, combinations, and simplifications that do not deviate from the spirit and principles of the present application shall be equivalent replacements and are included within the protection scope of the present application.

## Claims

1. Collagen, comprising the following amino acid sequence:
(1) an amino acid sequence shown in SEQ ID NO: 1;
(2) an amino acid sequence having 70%, 75%, 80%, 85%, 90% or 95% sequence identity to the amino acid sequence shown in SEQ ID NO: 1; or
(3) an amino acid sequence in which a mutation of one or more amino acid residues is made in the amino acid sequence shown in SEQ ID NO: 1; preferably, the mutation is a substitution, insertion, deletion or addition; preferably, the substitution is a conservative amino acid substitution;
preferably, the collagen is derived from human;
preferably, the collagen has a triple-helix structure, preferably a flexible triple-helix structure, preferably a 164.88° triple-helix structure; preferably, the collagen is in the form of a trimer;
preferably, the collagen has one or more of the following activities or effects: cell adhesion activity, cell migration-promoting activity, transdermal absorption, anti-wrinkle effect, sebum control effect, skin-restoring effect, and skin-soothing effect.

2. A nucleic acid encoding the collagen according to claim 1; wherein preferably, the nucleic acid has a nucleotide sequence shown in SEQ ID NO: 2.

3. A vector comprising the nucleic acid according to claim 2, optionally comprising a nucleotide encoding a purification tag, a nucleotide encoding a leader and/or a regulatory element;
wherein preferably, the purification tag is selected from a His tag, GST tag, MBP tag, SUMO tag, or NusA tag;
preferably, the regulatory element is selected from a promoter, terminator, and/or enhancer.

4. A host cell comprising a nucleic acid according to claim 2 or a vector according to claim 3; wherein preferably the host cell is a eukaryotic or prokaryotic cell; wherein preferably the eukaryotic cell is a yeast cell, animal cell and/or insect cell, and/or the prokaryotic cell is an *Escherichia coli* cell, such as *E. coli* BL21.

5. A method for producing the collagen according to claim 1, comprising:
(1) culturing the host cell according to claim 4 under a suitable condition;
(2) harvesting the host cell and/or medium containing the collagen; and
(3) purifying the fusion protein, for example, comprising (i) performing crude purification of the collagen using a Ni affinity chromatography column; (ii) adding collagenase for enzymatic cleavage; and/or (iii) performing fine purification of the collagen using an ion exchange column.

6. A composition comprising the collagen according to claim 1, the nucleic acid according to claim 2, the vector according to claim 3 and/or the host cell according to claim 4;
preferably, the composition is a pharmaceutical composition, food composition, or cosmetic composition,
preferably, the composition is one or more of the following: biological dressings, human body bionic materials, plastic surgery and beauty materials, organoid culture materials, cardiovascular stent materials, coating materials, tissue injection filler materials, ophthalmic materials, obstetrics and gynecology biomaterials, nerve repair and regeneration materials, liver tissue materials as well as vascular repair and regeneration materials, 3D-printed artificial organ biomaterials, cosmetic raw materials, pharmaceutical excipients, and food additives;
preferably, the cosmetic composition is a cosmetic composition with anti-wrinkle effect, sebum control effect, skin-restoring effect and/or skin-soothing effect; preferably, the sebum control effect is the sebum control effect on face;
preferably, the composition comprises a pharmaceutically, cosmetically and/or food acceptable carrier;
preferably, the composition is a solid, liquid or gel composition;
preferably, the composition is a solution dosage form, a lyophilized powder dosage form, a gel dosage form, a sponge dosage form or a fiber dosage form;
preferably, the composition is a composition for oral and/or topical administration; preferably, the topical administration is a topical administration to the skin; preferably, the composition is a transdermal composition;
preferably, the composition is a kit;
preferably, the composition is a liquid formulation, which comprises the collagen according to claim 1 and a pharmaceutically, cosmetically and/or food acceptable carrier; preferably, the carrier is a buffer, such as a D-PBS buffer or a PBS buffer.

7. A method for promoting cell adhesion or attachment, comprising contacting the cell with the collagen according to claim 1 and/or the composition according to claim 6.

8. A method for promoting cell migration, comprising contacting the cell with the collagen according to claim 1 and/or the composition according to claim 6.

9. A cosmetic or beauty method, comprising administering the collagen according to claim 1 and/or the composition according to claim 6 to the skin, and optionally applying another beauty and/or cosmetic product to the skin;
wherein preferably, the cosmetic or beauty method is a method for delivering anti-wrinkle, sebum control, skin restoration, and/or skin-soothing treatments;
preferably, the administration is topical administration;
preferably, the administration is transdermal administration.

10. Use of the collagen according to claim 1, the nucleic acid according to claim 2, the vector according to claim 3 and/or the host cell according to claim 4 in a medical, cosmetic, or beauty product or for delivering anti-wrinkle, sebum control, skin restoration, and/or skin-soothing treatments,
preferably, the product is an external use product,
preferably, the cosmetic or beauty product is an anti-wrinkle, sebum control, skin-restoring, and/or skin-soothing product;
preferably, the product is selected from biological dressings, human body bionic materials, plastic surgery and beauty materials, organoid culture materials, cardiovascular stent materials, coating materials, tissue injection filler materials, ophthalmic materials, obstetrics and gynecology biomaterials, nerve repair and regeneration materials, liver tissue materials as well as vascular repair and regeneration materials, or 3D-printed artificial organ biomaterials.

11. Use of the collagen according to claim 1, the nucleic acid according to claim 2, the vector according to claim 3 and/or the host cell according to claim 4 in the manufacture of a medicament or kit for anti-inflammation or reducing an inflammatory cytokine such as TNF-α.

12. A method for reducing the level of an inflammatory cytokine in a cell, comprising contacting the cell with the collagen according to claim 1 and/or the composition according to claim 6.
